# EUROPEAN PATENT SPECIFICATION

(11) **EP 2 823 036 B1**
(45) Date of publication and mention of the grant of the patent: **17.04.2019**
(21) Application number: 13707618.8
(22) Date of filing: 04.03.2013
(51) Int. Cl.: C12N 5/0735, C12N 5/071, G01N 33/50

(54) **METHOD OF DETERMINING TERATOGENIC RISK**
VERFAHREN ZUR BESTIMMUNG DES TERATOGENEN RISIKOS
PROCÉDÉ DE DÉTERMINATION DE RISQUES TÉRATOGÈNES

(30) Priority: 07.03.2012 US 201261607622 P; 06.07.2012 US 201261668489 P
(43) Date of publication of application: 14.01.2015
(73) Proprietor: F. Hoffmann-La Roche AG, 4070 Basel (CH)
(72) Inventor: CHIAO, Eric, Chatham, NJ 07928 (US); KAMEOKA, Sei, Cambridge, MA 02142-1183 (US); KOLAJA, Kyle L., Montclair, NJ 07042 (US)
(74) Representative: Townsend, Carolin
(86) International application number: PCT/EP2013/054244
(87) International publication number: WO 2013/131841

(56) References cited:
- WO-A1-2010/149346
- WO-A2-2008/107912
- US-A1- 2006 003 313
- US-A1- 2011 287 974
- ADLER ET AL: "First steps in establishing a developmental toxicity test method based on human embryonic stem cells", TOXICOLOGY IN VITRO, ELSEVIER SCIENCE, GB, vol. 22, no. 1, 19 December 2007 (2007-12-19), pages 200-211, XP022393683, ISSN: 0887-2333, DOI: 10.1016/J.TIV.2007.07.013
- MEHTA A ET AL: "Assessment of drug induced developmental toxicity using human embryonic stem cells", CELL BIOLOGY INTERNATIONAL, ACADEMIC PRESS, GB, vol. 32, no. 11, 1 November 2008 (2008-11-01), pages 1412-1424, XP025574246, ISSN: 1065-6995, DOI: 10.1016/J.CELLBI.2008.08.012 [retrieved on 2008-08-20]
- ANNA M WOBUS ET AL: "Present state and future perspectives of using pluripotent stem cells in toxicology research", ARCHIVES OF TOXICOLOGY, SPRINGER-VERLAG, BERLIN, DE, vol. 85, no. 2, 12 January 2011 (2011-01-12), pages 79-117, XP019876617, ISSN: 1432-0738, DOI: 10.1007/S00204-010-0641-6
- SPIELMANN H ET AL: "THE EMBRYONIC STEM CELL TEST, AN IN VITRO EMBRYOTOXICITY TEST USING TWO PERMANENT MOUSE CELL LINES: 3T3 FIBROBLASTS AND EMBRYONIC STEM CELLS", IN VITRO TOXICOLOGY, MARY ANN LIEBERT, NEW YORK, NY, US, vol. 10, no. 1, 1 January 1997 (1997-01-01), pages 119-127, XP009044393, ISSN: 0888-319X
- DERK TEN BERGE ET AL: "Wnt Signaling Mediates Self-Organization and Axis Formation in Embryoid Bodies", CELL STEM CELL, vol. 3, no. 5, 1 November 2008 (2008-11-01), pages 508-518, XP55297343, AMSTERDAM, NL ISSN: 1934-5909, DOI: 10.1016/j.stem.2008.09.013

## Description

### FIELD OF THE INVENTION

The present invention relates to a method of determining the risk of drug induced teratogenicity using human pluripotent stem cells.

### BACKGROUND OF THE INVENTION

Improved *in vitro* systems for predicting drug-induced toxicities are needed by the pharmaceutical and biotechnology industries to decrease late-stage drug attrition. Specifically, there is a need for predictive high-throughput *in vitro* models that employ human cells and can reduce our reliance on animal studies.

One of the most devastating adverse drug effects that can occur is teratogenicity. Unfortunately, predicting whether a drug has the potential to cause fetal harm in humans is extremely difficult and primarily depends on the use of pregnant animal models. Currently, due to a lack of robust alternative methods, the FDA requires several phases of animal studies to assess the risk of reproductive harm (Bailey, G. P., L. D. Wise, et al. (2009), "Pre- and postnatal developmental toxicity study design for pharmaceuticals." Birth Defects Research Part B: Developmental and Reproductive Toxicology 86(6): 437-445). However, the dependence on animal systems runs the risk of failing to identify human teratogens such as thalidomide, that are harmful to human fetal development, but not other animal species such as rodents (Shuey, D. and J. H. Kim (2011), "Overview: developmental toxicology-new directions." Birth Defects Research Part B: Developmental and Reproductive Toxicology, 92(5): 381-383).

Pluripotent stem cells (PSCs) can be grown indefinitely in a dish while maintaining a "blank" or "undifferentiated" stem cell state. Then, by changing the growth conditions, the cells can be directed to "differentiate" into all the tissues of the human body. During the "differentiation" process, the cells are regulated by the normal genetic programs that control fetal development. A method for characterizing compounds for teratogenic risk using pluripotent mouse embryonic stem (ES) cells has been pioneered by the laboratory of Hans Spielman (Spielmann, H., I. Pohl, et al. (1997), "The embryonic stem cell test (EST), an in vitro embryotoxicity test using two permanent mouse cell lines: 3T3 fibroblasts and embryonic stem cells." In vitro Toxicology 10: 119-127). This method, referred to as the Embryonic Stem Cell Test, or EST, involves differentiating mouse ES cells for ten days until beating cardiomyocytes can be observed. The teratogenic risk is assessed by the use of a biostatistical prediction model that compares the concentrations of drug that exhibits 50% inhibition differentiation and 50% cytotoxicity of the mouse ES cells and a mouse differentiated fibroblast line (Seiler, A. E. and H. Spielmann (2011), "The validated embryonic stem cell test to predict embryotoxicity in vitro." Nat Protoc 6(7): 961-78). However, this method suffers from many limitations such as the relatively long length of the assay, the labor-intensive methods required for differentiating the cells, the qualitative nature of scoring "beating" cardiomyocytes, and moderate accuracy of the biostatistical prediction model (Marx-Stoelting, P. E. Adriaens, et al. (2009), "A review of the implementation of the embryonic stem cell test (EST). The report and recommendations of an ECVAM/ReProTect Workshop." Altern Lab Anim 37(3): 313-28). Furthermore, the mouse EST fails to accurately detect known human teratogens such as thalidomide.

Due to these limitations, many independent labs have sought to develop an improved *in vitro* method for assessing teratogenic risk. Most of these efforts have been aimed at increasing the assay complexity, based on the assumption that only a more complex *in vitro* system would provide adequate predictivity of the vast biological complexity of *in vivo* gestation. For example, attempts to improve the mouse EST have included employing toxicogenomic analysis to the standard cardiomyocyte differentiation protocol (Hewitt, M., C. M. Ellison, et al. (2010), "Integrating (Q) SAR models, expert systems and read-across approaches for the prediction of developmental toxicity." Reproductive Toxicology 30(1): 147-160; van Dartel, D. A. M., J. L. A. Pennings, et al. (2010), "Monitoring Developmental Toxicity in the Embryonic Stem Cell Test Using Differential Gene Expression of Differentiation-Related Genes." Toxicological Sciences 116(1): 130-139; Pennings, J. L. A., D. A. M. van Dartel, et al. (2011), "Gene set assembly for quantitative prediction of developmental toxicity in the embryonic stem cell test." Toxicology 284(1-3): 63-71; van Dartel, D. A. M. and A. H. Piersma (2011). "The embryonic stem cell test combined with toxicogenomics as an alternative testing model for the assessment of developmental toxicity." Reproductive Toxicology 32(2): 235-244); adding additional differentiated cell types such as endothelial cells or bone cells to the assay (Festag, M., B. Viertel, et al. (2007), "An in vitro embryotoxicity assay based on the disturbance of the differentiation of murine embryonic stem cells into endothelial cells. II. Testing of compounds." Toxicology in Vitro 21(8): 1631-1640; Buesen, R., E. Genschow, et al. (2009), "Embryonic stem cell test remastered: comparison between the validated EST and the new molecular FACS-EST for assessing developmental toxicity in vitro." Toxicol Sci 108(2): 389-400; zur Nieden, N. I., L. A. Davis, et al. (2010), "Comparing three novel endpoints for developmental osteotoxicity in the embryonic stem cell test." Toxicology and Applied Pharmacology 247(2): 91-97); or assessing changes in metabolites secreted into the media during stem cell differentiation (West, P. R., A. M. Weir, et al. (2010), "Predicting human developmental toxicity of pharmaceuticals using human embryonic stem cells and metabolomics." Toxicology and Applied Pharmacology 247(1): 18-27). Although some of these methods have resulted in an increase in predictivity, this has often been at the expense of assay throughput.

WO2008/107912 describes in vitro assay methods for classifying embryotoxicity of compounds. WO2010149346 describes methods for predicting the toxicity of a chemical. US2011/287974 describes methods and kits for ascertaining biosafety of an agent. US2006/003313 describes methods for identifying factors for differentiating definitive endoderm. Adler et al (Toxicology in vitro 22(1): 200-211) describes first steps in establishing a developmental toxicity text based on human embryonic stem cells. Mehta et al (Cell biology international academic press 32(11:1412-1424) describes assessment of drug induced developmental toxicity using human embryonic stem cells. Wobus et al (Archives of toxicology 85(2):79-117) describes the present and future perspectives of using pluripotent stem cells in toxicology research. Spielmann et al (In vitro toxicology 10(1):119-127) describes the embryonic stem cell test, an in vitro embryotoxicity test using two permanent mounse cell lines. Ten Berge et al (Cell stem cell 3(5):508-518) describes that Wnt signaling meadiates self-organization and axis formation in embryoid bodies.

In contrast to the efforts mentioned above, the novel method described herein employs a seemingly counter-intuitive approach: shortening the stem cell differentiation to 2.5 days and examining mesendoderm lineage markers as the sole endpoint. Therefore this new approach interrogates a much earlier, transient stage of embryo development. Using human pluripotent stem cells, we demonstrate that this method achieves superior throughput and predictivity compared to existing methods.

### SUMMARY OF THE INVENTION

The present application provides a method of assessing the teratogenic risk of a compound comprising:
(1) contacting the compound with pluripotent stem cells that are being differentiated into mesendoderm under conditions that reproducibly achieve 40-60% mesendoderm formation in 3 days of differentiation;
(2) measuring the expression of one or more mesendodermal markers, wherein the one or more mesendodermal markers are selected from the group consisting of Sox17, EOMES or T-brachyury;
(3) determining the IC50 concentration of the compound which results in 50% inhibition of the protein expression of the one or more markers; and
(4) comparing the IC50 concentration determined in step (3) with the IC50 concentrations of:
   (a) one or more compounds with known teratogenic risks and (b) one or more compounds with known non-teratogenic risks, to establish a teratogenic risk of the compound being assessed,
wherein the pluripotent stem cells are human pluripotent stem cells and wherein at least one of the mesendodermal markers is SOX17.

### BRIEF DESCRIPTION OF THE DRAWINGS

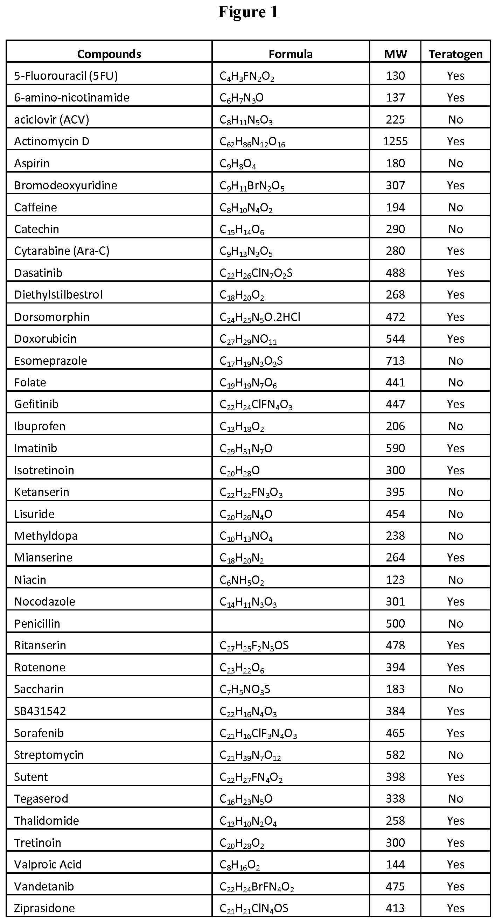

**Figure 1****.** Known compounds demonstrated to be either teratogens or non-teratogens *in vivo.*

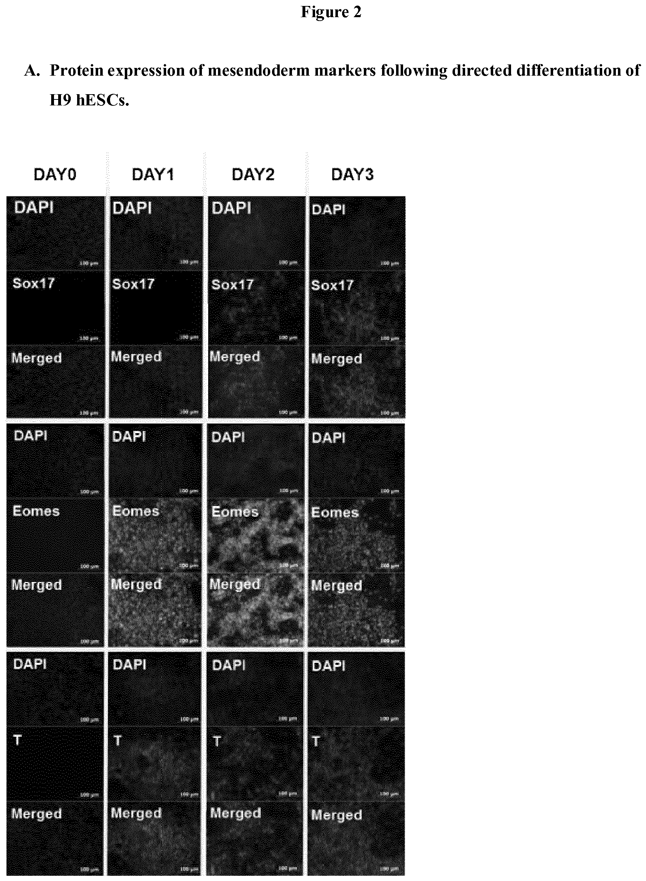

**Figure 2****.** A time course showing the reference H9 human embryonic stem cell line differentiated for 3 days as described in the **Protocol Methods.** At each day indicated, the cells were fixed and stained with antibodies specific to either Sox17, EOMES or T-brachyury. The DNA stain DAPI was used as a counterstain to mark cell nuclei.

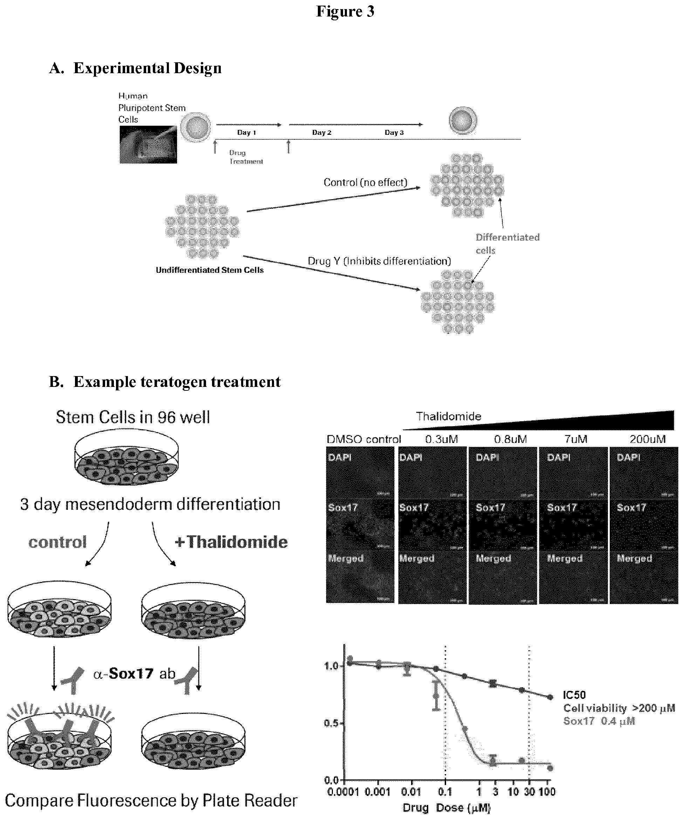

**Figure 3****.** Panel A: Experimental design graphically depicts the 3 day differentiation protocol used in the present invention. Panel B: An example of a teratogen treatment demonstrates the dose-dependent reduction of Sox17 staining.

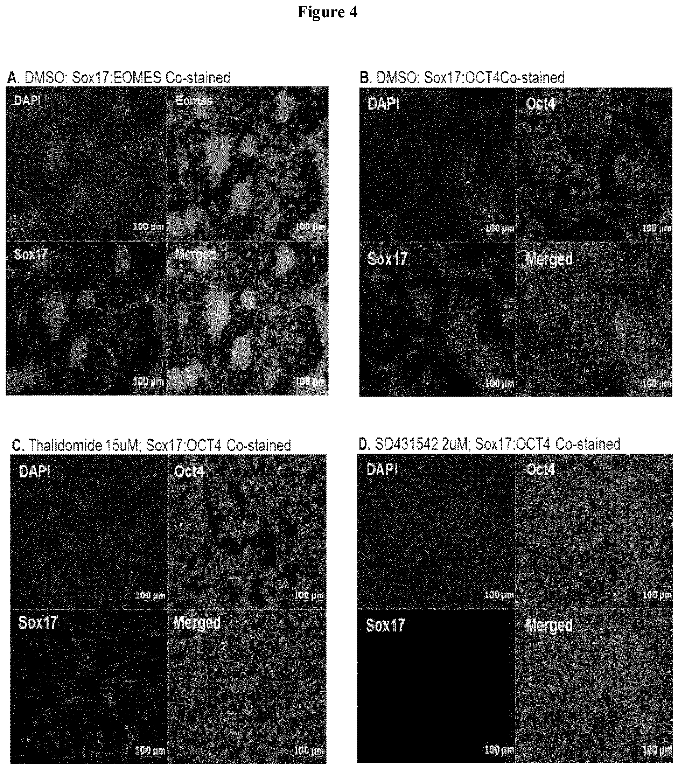

**Figure 4****.** Co-expression of the cell lineage markers Sox17, EOMES (mesendoderm markers) and OCT4 (a marker of pluripotent cells and neural lineages). Panel A shows the overlapping expression of Sox17 and EOMES after 3 days of differentiation, marking the mesendodermal lineage. Panel B shows the nuclear staining of Sox17 and OCT4 are largely mutually exclusive. Panels C and D illustrate teratogen induced relative reduction of Sox17 and increase in OCT4 staining, suggesting that relative expression of cell lineage markers other than Sox17 or EOMES may be used as measures of teratogenic risk.

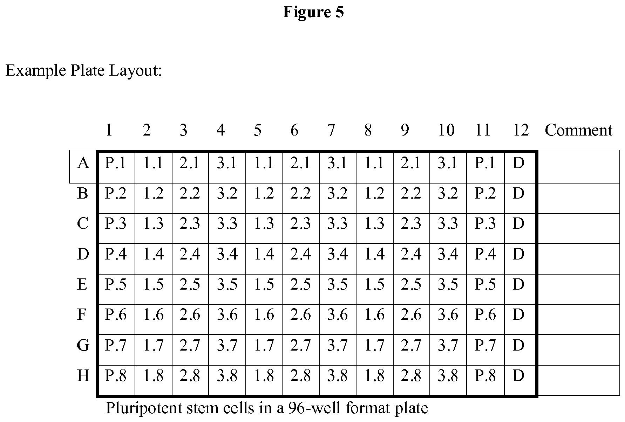

**Figure 5****.** An example 96-well plate layout for testing unknown compounds for teratogenic risk. The numbers in boxes refer to the compound in a certain concentration. For example 1.1 = compound 1 in concentration 1, 2.1 = compound 2 in concentration 1. In addition, P = positive control (with a known teratogen) and D = DMSO (the negative control).

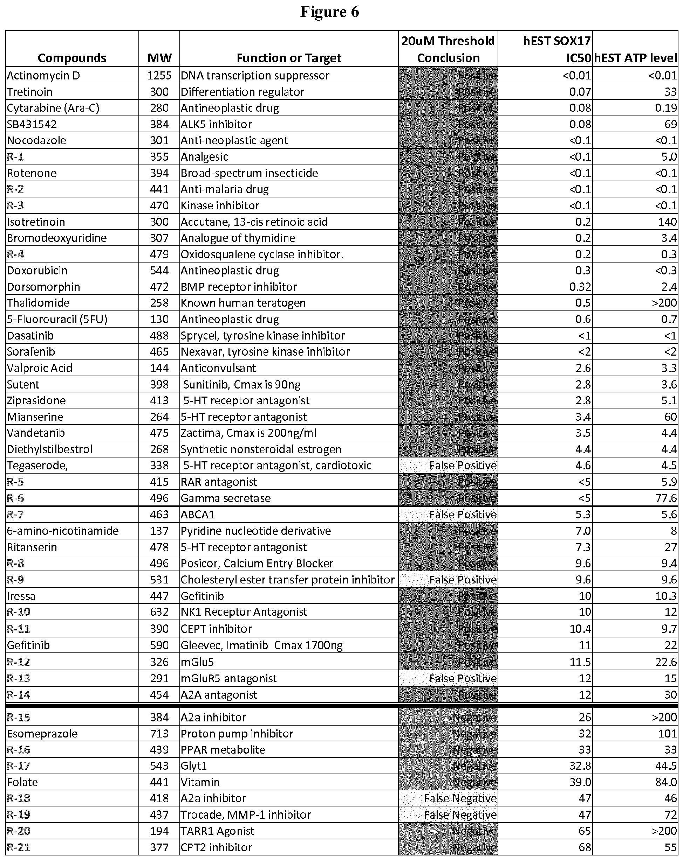

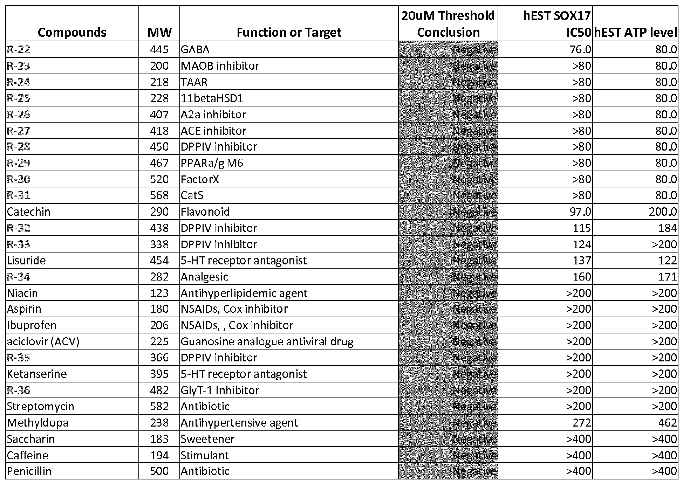

**Figure 6****.** The table depicts the experimentally determined Sox17 inhibition IC50 for 75 compounds known to be either teratogenic or non-teratogenic in vivo. Using these data, we were able to establish an IC50 cut-off of 20 uM that could distinguish teratogenic from non-teratogenic compounds with an accuracy of 92%.

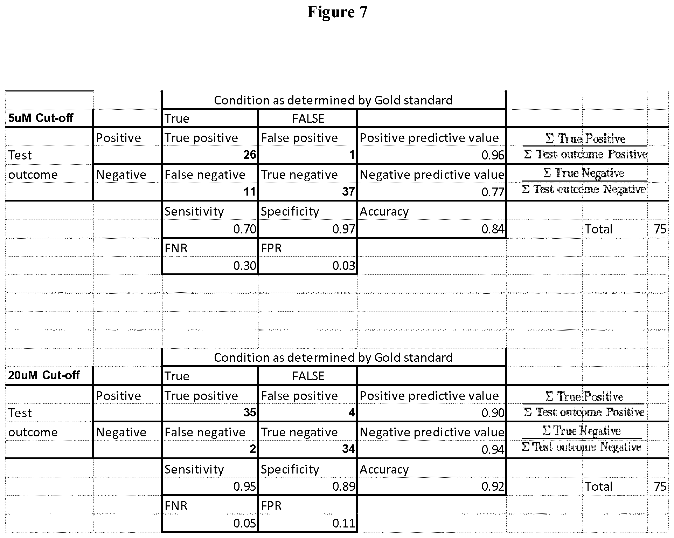

**Figure 7**. The table shows the predictive value calculated from the results in **Figure 6** based on IC50 values of either 5uM or 20uM. The exact IC50 cut-off used by an individual should be chosen based on their tolerance of the predicted rate of false positives versus false negatives.

### DETAILED DESCRIPTION OF THE INVENTION

As used in this specification, whether in a transitional phrase or in the body of the claim, the terms "comprise(s)" and "comprising" are to be interpreted as having an open-ended meaning. That is, the terms are to be interpreted synonymously with the phrases "having at least" or "including at least". When used in the context of a process, the term "comprising" means that the process includes at least the recited steps, but may include additional steps. When used in the context of a compound or composition, the term "comprising" means that the compound or composition includes at least the recited features or components, but may also include additional features or components.

The term "optional" or "optionally" as used herein means that a subsequently described event or circumstance may, but need not, occur, and that the description includes instances where the event or circumstance occurs and instances in which it does not.

The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%.

The term "pluripotent stem cell" as used herein means and includes the ability of a cell to differentiate into cell types of all three lineages or germ layers (viz. endoderm, ectoderm, and mesoderm). The term multipotent has a meaning understood in the art, and includes the ability of a cell to differentiate into multiple cell types. It is also understood that multipotent cells may be more restricted in their ability to differentiate than pluripotent cells. The term induced stem cells ("iSCs"), as used herein, refer to induced pluripotent stem cells ("iPSCs") or to induced multipotent stem cells ("iMSCs"). At times, the term "iPS" or "iPS cell" may be used instead of "iPSC"; similarly, at times the term "iMS" or "iMS cell" may be used instead of "iMSC". The methods and compositions described herein that are applicable to iPSCs are also applicable to iSCs and iMSCs.

The term "measuring the protein expression" means determining the quantity of protein produced (e.g., in the well of cells by either direct methods such as antibody staining and quantifying the specific fluorescence of the antibody stained cells or indirectly by measuring the amount of mRNA encoding the protein).

The term "IC 50" as used herein means the concentration of a test compound that results in 50% inhibition of the measured differentiation marker relative to an untreated control.

The term "developmentally regulated markers" means genes whose expression change during development.

The term the "mesendodermal markers" means genes whose expression has been determined to be found in the mesoderm cell lineage. Examples of mesendodermal markers useful in this invention are Sox 17, EOMES, and T-brachyury.

The term "teratogenic risk" means the calculated risk or probability that a compound will be teratogenic in vivo. Similarly, the term "non-teratogenic risk" means the calculated risk or probability that a compound will not be teratogenic in vivo.

The term "Sox17" refers to the gene or gene product encoded by SRY-box 17 (SOX17, gene ID 64321, the gene product of the human homologue is exemplified in SEQ ID NO:1).

The term "EOMES" refers to the gene or gene product encoded by eomesodermin (EOMES, gene ID 8320, the gene product of the human homologue is exemplified in SEQ ID NO:2).

The term "T-brachyury" refers to gene or gene product encoded by T, brachyury homolog (mouse) (T, gene ID 6862 the gene product of the human homologue is exemplified in SEQ ID NO:3).

The term OCT4 refers to gene or gene product encoded by POU5F1 POU class 5 homeobox 1 (POU5F1, gene ID: 5460 the gene product of the human homologue is exemplified in SEQ ID NO:4).

The term "high throughput" as used herein means an assay design that allows easy screening of multiple samples simultaneously including capacity for robotic manipulation. Another desired feature of high throughput assays is an assay design that is optimized to reduce reagent usage, or minimize the number of manipulations in order to achieve the analysis desired. Examples of assay formats include 96-well or 384-well plates. It is well known in the art that as miniaturization of plastic molds and liquid handling devices are advanced, or as improved assay devices are designed, greater numbers of samples may be performed using the design of the present invention. In one embodiment, the cells are cultured and analyzed in the micro-titer plates containing a plurality of wells such as 96- or 386-well plates.

Technical and scientific terms used herein have the meaning commonly understood by one of skill in the art to which the present invention pertains, unless otherwise defined. Reference is made herein to various methodologies and materials known to those of skill in the art. Standard reference works setting forth the general principles of pharmacology include Goodman and Gilman's The Pharmacological Basis of Therapeutics, 10th Ed., McGraw Hill Companies Inc., New York (2001). Any suitable materials and/or methods known to those of skill can be utilized in carrying out the present invention. However, preferred materials and methods are described. Materials, reagents and the like to which reference are made in the following description and examples are obtainable from commercial sources, unless otherwise noted.

The present application discloses a method of assessing the teratogenic risk of a compound comprising:
(1) contacting the compound with pluripotent stem cells that are being differentiated into mesendoderm;
(2) detecting the progression of embryonic development by measuring the protein expression of one or more developmentally regulated markers;
(3) determining the IC50 concentration of the compound which results in 50% inhibition of the protein expression of the one or more markers; and
(4) comparing the IC50 concentration determined in step (3) with the IC50 concentrations of:
   (a) one or more compounds with known teratogenic risks and (b) one or more compounds with known non-teratogenic risks, to establish a teratogenic risk of the compound being assessed.

In particular embodiments, the protein expression of one or more mesendodermal markers are measured.

In more particular embodiments, the protein expression of one or more of the mesendodermal markers measured are selected from the group consisting of Sox 17, EOMES, and T-brachyury.

In particular, the present invention provides a method of assessing the teratogenic risk of a compound comprising:
(1) contacting the compound with pluripotent stem cells that are being differentiated into mesendoderm under conditions that reproducibly achieve 40-60% mesendoderm formation in 3 days of differentiation;
(2) measuring the protein expression of one or more mesendodermal markers at day 3 of differentiation, wherein the one or more mesendodermal markers are selected from the group consisting of Sox17, EOMES, and T-brachyury;
(3) determining the IC50 concentration of the compound which results in 50% inhibition of the protein expression of the one or more markers; and
(4) comparing the IC50 concentration determined in step (3) with the IC50 concentrations of:
   (a) one or more compounds with known teratogenic risks and (b) one or more compounds with known non-teratogenic risks, to establish a teratogenic risk of the compound being assessed
wherein the pluripotent stem cells are human pluripotent stem cells and wherein at least one of the mesendodermal markers is SOX17.

In particular embodiments, the protein expression of at least one mesendodermal marker measured is Sox 17; and in more particular the protein expression of SOX17 is the only mesendodermal marker measured.

In other particular embodiments, the protein expression of at least one mesendodermal marker measured is EOMES; and in more particular the protein expression of EOMES is the only mesendodermal marker measured.

In other particular embodiments, the protein expression of at least one mesendodermal marker measured is T-brachyury; and in more particular the protein expression of T-brachury is the only mesendodermal marker measured.

In other particular embodiments, the protein expression of SOX17 and EOMES are the only mesendodermal markers measured.

In other particular embodiments, the protein expression of SOX17 and T-brachyury are the only mesendodermal markers measured.

In particular embodiments, the pluripotent stem cells are of primate origin.

In particular embodiments, the pluripotent stem cells are from an embryonic stem cell source.

In other particular embodiments, the pluripotent stem cells are from an induced pluripotent stem cell source.

In particular embodiments, the teratogenic risk is assessed by quantitating changes in the protein expression of markers by immuno-histochemistry.

In other particular embodiments, the teratogenic risk is assessed by quantitating changes in the protein expression of markers by flow cytometry.

In other particular embodiments, the teratogenic risk is assessed by quantitating changes in the protein expression of markers by fluorescence microscopy.

In other particular embodiments, the teratogenic risk is assessed by quantitating changes in the protein expression of markers by quantitating changes in the mRNA levels encoding the protein markers.

In particular embodiments, step (1) of the method of the present invention is performed after 40-60% of said pluripotent stem cells are differentiated into mesendoderm.

In particular embodiments, the one or more compounds with known teratogenic risks in step (4) are selected from the group consisting of known teratogens listed in Figure 1.

In other particular embodiments, the one or more compounds with known teratogenic risks in step (4) are selected from the group consisting of known teratogens listed in Figure 6.

In particular embodiments, the one or more compounds with known non-teratogenic risks in step (4) are selected from the group consisting of known teratogens listed in Figure 1.

In other particular embodiments, the one or more compounds with known non-teratogenic risks in step (4) are selected from the group consisting of known teratogens listed in Figure 6.

In more particular embodiments, the one or more compounds with known teratogenic risks in step (4) are selected from the group consisting of: actinomycin D, tretinoin, cytarabine, nocodazole, rotenone, tretinoin, isotretinoin, bromodeoxyuridine, doxorubicin, dorsomorphin, thalidomide, 5-Fluorouracil, dasatinib, sorafenib, valproic acid, sunitinib, ziprasidone, mianserine, vandetanib, diethylstilbestrol, 6-amino-nicotinamide, ritanserin, and gefitinib.

In further particular embodiments, the one or more compounds with known teratogenic risks in step (4) are selected from the group consisting of: thalidomide, mianserine, and ritanserin.

In particular embodiments, the one or more compounds with known non-teratogenic risks in step (4) are selected from the group consisting of: esomeprazole, folate, catechin, lisuride, niacin, aspirin, ibuprofen, acyclovir, ketanserine, streptomycin, methyldopa, saccharin, caffeine, penicillin, and tegaserod.

In more particular embodiments, the one or more compounds with known non-teratogenic risks in step (4) are selected from the group consisting of: folate and methyldopa.

In more specific embodiments, the one or more compounds with known teratogenic risks in step (4) are selected from the group consisting of: thalidomide, mianserine, and ritanserin and the one or more compounds with known non-teratogenic risks are selected from the group consisting of folate and methyldopa.

In one embodiment said method is an in vitro method.

In one aspect of the disclosure there is provided a method of assessing the teratogenic risk of a compound comprising:
(1) contacting the compound with pluripotent stem cells that are being differentiated into mesendoderm;
(2) measuring the protein expression of one or more mesendodermal markers selected from the group consisting of Sox17, EOMES, and T-brachyury;
(3) determining the IC50 concentration of the compound which results in 50% inhibition of the protein expression of said one or more markers; and
(4) comparing the IC50 concentration determined in step (3) with the IC50 concentrations of:
   (a) one or more compounds with known teratogenic risks and (b) one or more compounds with known non-teratogenic risks, to establish a teratogenic risk of the compound being assessed.

In one embodiment, the pluripotent stem cells are of primate origin.

In one embodiment, the pluripotent stem cells are embryonic stem cells.

In one aspect of the disclosure, the pluripotent stem cells are H9 human embryonic stem cells.

In one embodiment, the pluripotent stem cells are induced pluripotent stem cells.

In one embodiment, at least one of the mesendodermal markers is SOX17.

In one embodiment, at least one of the mesendodermal markers is EOMES.

In one embodiment, at least one of the mesendodermal markers is T-brachyury.

In one embodiment, the protein expression of SOX17 is the only mesendodermal marker measured.

In one embodiment, the protein expression of SOX17 and EOMES are the only mesendodermal markers measured.

In one embodiment, the protein expression of SOX17 and T-brachyury are the only mesendodermal markers measured.

In one embodiment, the protein expression is measured by immuno-histochemistry.

In one embodiment, the protein expression is measured by flow cytometry.

In one embodiment, the protein expression is measured by fluorescence microscopy.

In one embodiment, there is provided a method of assessing the teratogenic risk of a compound comprising:
(1) contacting the compound with pluripotent stem cells that are being differentiated into mesendoderm;
(2) measuring the protein expression of one or more mesendodermal markers selected from the group consisting of Sox17, EOMES, and T-brachyury;
(3) determining the IC50 concentration of the compound which results in 50% inhibition of the protein expression of said one or more markers; and
(4) comparing the IC50 concentration determined in step (3) with the IC50 concentrations of:
   (a) one or more compounds with known teratogenic risks and (b) one or more compounds with known non-teratogenic risks, to establish a teratogenic risk of the compound being assessed,
wherein step (1) is performed after 40-60% of said pluripotent stem cells are differentiated into mesendoderm.

In one aspect, there is described a method of assessing the teratogenic risk of a compound comprising:
(1) contacting the compound with pluripotent stem cells that are being differentiated into mesendoderm;
(2) measuring the protein expression of one or more mesendodermal markers selected from the group consisting of Sox17, EOMES, and T-brachyury;
(3) determining the IC50 concentration of the compound which results in 50% inhibition of the protein expression of said one or more markers; and
(4) comparing the IC50 concentration determined in step (3) with the IC50 concentrations of:
   (a) one or more compounds with known teratogenic risks and (b) one or more compounds with known non-teratogenic risks, to establish a teratogenic risk of the compound being assessed,

wherein the one or more compounds with known teratogenic risks in step (4) are selected from the group consisting of: actinomycin D, tretinoin, cytarabine, nocodazole, rotenone, tretinoin, isotretinoin, bromodeoxyuridine, doxorubicin, dorsomorphin, thalidomide, 5-Fluorouracil, dasatinib, sorafenib, valproic acid, sunitinib, ziprasidone, mianserine, vandetanib, diethylstilbestrol, 6-amino-nicotinamide, ritanserin, and gefitinib.

In one aspect, there is described a method of assessing the teratogenic risk of a compound comprising:
(1) contacting the compound with pluripotent stem cells that are being differentiated into mesendoderm;
(2) measuring the protein expression of one or more mesendodermal markers selected from the group consisting of Sox17, EOMES, and T-brachyury;
(3) determining the IC50 concentration of the compound which results in 50% inhibition of the protein expression of said one or more markers; and
(4) comparing the IC50 concentration determined in step (3) with the IC50 concentrations of:
   (a) one or more compounds with known teratogenic risks and (b) one or more compounds with known non-teratogenic risks, to establish a teratogenic risk of the compound being assessed,
wherein the one or more compounds with known teratogenic risks in step (4) are selected from the group consisting of: thalidomide, mianserine, and ritanserin.

In one aspect, there is described a method of assessing the teratogenic risk of a compound comprising:
(1) contacting the compound with pluripotent stem cells that are being differentiated into mesendoderm;
(2) measuring the protein expression of one or more mesendodermal markers selected from the group consisting of Sox17, EOMES, and T-brachyury;
(3) determining the IC50 concentration of the compound which results in 50% inhibition of the protein expression of said one or more markers; and
(4) comparing the IC50 concentration determined in step (3) with the IC50 concentrations of:
   (a) one or more compounds with known teratogenic risks and (b) one or more compounds with known non-teratogenic risks, to establish a teratogenic risk of the compound being assessed,
wherein the one or more compounds with known non-teratogenic risks in step (4) are selected from the group consisting of: esomeprazole, folate, catechin, lisuride, niacin, aspirin, ibuprofen, acyclovir, ketanserine, streptomycin, methyldopa, saccharin, caffeine, penicillin, and tegaserod.

In one aspect, there is described a method of assessing the teratogenic risk of a compound comprising:
(1) contacting the compound with pluripotent stem cells that are being differentiated into mesendoderm;
(2) measuring the protein expression of one or more mesendodermal markers selected from the group consisting of Sox17, EOMES, and T-brachyury;
(3) determining the IC50 concentration of the compound which results in 50% inhibition of the protein expression of said one or more markers; and
(4) comparing the IC50 concentration determined in step (3) with the IC50 concentrations of:
   (a) one or more compounds with known teratogenic risks and (b) one or more compounds with known non-teratogenic risks, to establish a teratogenic risk of the compound being assessed,
wherein the one or more compounds with known non-teratogenic risks in step (4) are selected from the group consisting of: folate and methyldopa.

In one aspect, there is discribed a method of assessing the teratogenic risk of a compound comprising:
(1) contacting the compound with pluripotent stem cells that are being differentiated into mesendoderm;
(2) measuring the protein expression of one or more mesendodermal markers selected from the group consisting of Sox17, EOMES, and T-brachyury;
(3) determining the IC50 concentration of the compound which results in 50% inhibition of the protein expression of said one or more markers; and
(4) comparing the IC50 concentration determined in step (3) with the IC50 concentrations of:
   (a) one or more compounds with known teratogenic risks and (b) one or more compounds with known non-teratogenic risks, to establish a teratogenic risk of the compound being assessed,
wherein the one or more compounds with known teratogenic risks in step (4) are selected from the group consisting of: thalidomide, mianserine, and ritanserin; and
the one or more compounds with known non-teratogenic risks are selected from the group consisting of folate and methyldopa.

### EXAMPLES

### General Method of Determining Teratogenic Rick

The method of the present invention assesses the risk of drug induced teratogenicity by comparing the levels of mesendoderm formation in treated versus control pluripotent stem cells undergoing directed differentiation. Therefore, one must first establish the optimal conditions for directing the differentiation of the human pluripotent stem cells towards mesendoderm. As a proof of concept, an initial validation was performed using as reference the H9 human embryonic stem cell line using the protocol described in the Examples herein.

### Optimizing the baseline differentiation

First, the optimal initial cell plating density and concentration of growth factors that reproducibly achieved 40-60% mesendoderm formation in 3 days of differentiation was identified (see **Figure 2**). With minor protocol optimizations of the ideal concentrations and duration of growth factor treatments to generate mesendoderm, a person skilled in the art could adapt this method for use with most human pluripotent cell lines. Although 40-60% differentiation was chosen as the starting point for these studies, since the method relies on comparing the ratio of mesendoderm formation in drug treated samples versus control samples, the exact percent mesendoderm cells should not be critical as long as the efficiency is consistent from well to well.

During the course of the initial validation experiments, a variety of lineage markers, antibodies, and quantitation methods were examined. Those experiments showed that the co-expressed mesendoderm lineage markers Sox17, T-brachyury and EOMES were informative for monitoring developmental disruptions. One would also predict that the quantiation of other markers that label the Sox17/EOMES/T-brachyury negative cells, such as OCT4 as shown in **Figure 4** could similarly be used to quantitate the disruption of the signal transduction pathways guiding the directed differentiation.

### Establishing the threshold concentration for in vitro/in vivo correlation

After optimizing conditions for the directed differentiation, the dose-response relationship that permits stratification of known teratogenic compounds from compounds known to not cause fetal harm is established. For the initial studies we used a panel of 30 commercially available compounds and 40 proprietary compounds with known *in vivo* effects. Individual wells containing cells being differentiated towards mesendoderm were treated on day 1 and day 2 (see **Figure 3**). On a single plate, increasing concentrations of each compound were applied to a unique well. In addition, each plate contained wells treated with compounds known to be teratogenic as a positive control and also wells treated with only solvent as a negative control. (See example plate-map in **Figure 5**).

At the conclusion of the directed differentiation, the levels of mesendoderm lineage markers were quantitated. For the proof of principle studies described herein, the expression of the lineage markers in the cells was measured at the protein level by immune-staining, followed by quantification by either flow cytometry analysis, fluorescence microscopy or a using a plate reader. From these values, the concentration of each test compound that resulted in 50% inhibition of the measured differentiation marker relative to the DMSO control was calculated (the IC50) for each test compound. Examining IC50 values of the compounds with known to be either teratogenic or non-teratognic *in vivo* allows one to establish the IC50 values that distinguish teratogenic compounds from non-teratogenic compounds. For the reference H9 cell line examined (below) during the validation, it was found that a concentration of 20uM provided >90% accuracy for predicting risk of teratogencity (see tables in **Figures 6** and **7**).

### Adapting the assay for high-throughput screening

Once the desired threshold for measuring teratogenic risk is established using the pluripotent cell line of interest, the assay can be employed for higher-throughput risk assessments. Compounds can be tested in triplicate at a single concentration that correlates to a level of acceptable predictivity.

### Specific Protocol & Method For The Compounds Tested

### Basic Reagents

**Reagents**

16% Formaldehyde Solution, 10x10ml ampule, Thermo, #28908
30% Albumin Solution from bovine serum, Sigma, catalog #A9576-50ML
100ml Donkey Serum, Millipore, #S30-100ML
Human SOX17 NL557 Affinity Purified Polyclonal Ab, Goat IgG for immunohistochemistry R&D systems, Inc. #NL1924R
hESC-qualified Matrigel, 5 ml *LDEV-Free, BD Bioscience, #354277, extracellular matrix Recombinant Human/Mouse/Rat Activin A, R&D Systems, Inc. #338-AC-005
Recombinant Human Wnt-3a, R&D Systems, Inc. #5036-WN-010
EmbryoMax® ES Cell Qualified Fetal Bovine Serum, 500ml, Millipore, #ES009B (ES-009-B)
ACCUTASE™ cell detachment solution, Stemcell Technologies, #07920, 100 mL
mTeSR®1, StemCell Technologies, #05850 1 Kit, defined human pluripotent stem cell liquid media
Y-27632 dihydrochloride monohydrate ((*R*)-(+)-*trans*-4-(1-Aminoethyl)-N-(4-Pyridyl)cyclohexanecarboxamide dihydrochloride, Tocris,
Advanced RPMI Medium 1640 (IX), liquid, No Glutamine Phenol Red 500 ml,
Invitrogen, #12633-012, basel cell growth media
Glutamine 100x #25030-081 from Invitrogen
Image-iT™ FX Signal Enhancer, Invitrogen, #136933 (optional), fluorescence stain signal enhancer
Triton X-100, Sigma, #T8787
SlowFade® Gold antifade reagent with DAPI, Invitrogen, #S36938 fluorescence stain signal preservative
Dimethyl sulfoxide (DMSO), #494429 - Sigma
DE diff medium-I (MAKE afresh on the same day):
Advanced RPMI Medium 1640
Glutamine (1x, add fresh)
human Activin A 100ng/ml
human Wnt3a 25ng/ml.
DE diff medium-II (MAKE afresh on the same day):
Advanced RPMI Medium 1640
Glutamine (1x, add fresh)
human Activin A 100ng/ml
0.1% Fetal Bovine Serum (FBS).
Matrtigel coating of tissue culture plates:
Coat 96 well with Matrigel using a 1:80 dilution.

### Protocol Methods

### Pluripotent stem cell preparation:

**Cell Culture.** Reference H9 human ES cells (From WiCell) were thawed in mTeSR medium with 10uM Y-27632. Plate 3 millions cells in two 100mm tissue culture grade dishes pre-coated with ES cell qualified Matrigel (BD Biosciences). Y-27632 is required only during seeding and it should be removed within 24 hours after seeding. Change mTeSR media every day. Cells should be near-confluent within 3-4 days.

### ACCUTASE and SEED ON 96 WELL PLATES.

When cells become about 50% confluent remove media, rinse with PBS, then add 2ml of accutase per 100mm plate. Cells should detach within 3 minutes. Immediately add 8ml mTeSR with 10uM Y-27632, transfer 20ml (from 2x 100mm plate) to 50ml tubes. Spin at 400xg for 6 min. Remove supernatant. Add 10ml mTeSR with Y-27632. Pipet up and down to suspend cell pellet. Count the number of viable cells and re-suspend to 1 millions cells/ml. Seed cells at between 5000-50,000 cells/96 well. Depending on the specific cell line and lot of cells, this number may vary in order to attain optimal cell density. For these experiments, 15,000 cells/well were plated. Change mTESR media every day for 2-5 days.

### Pluripotent Stem Cell Directed Differentiation and Analysis

Day 0. START DIFFERENTIATION with Diff Media-I with Drug Cells should be evenly distributed in 96 well plates. Begin differentiation when cells are about 35% confluency in the wells of 96 well plate (after 2-5 days). Remove media, and wash it with Advanced RPMI 4-5 times. It's important to wash away mTESR completely, as it contains very high concentration of various factors that maintain the cells in the undifferentiated state. Add DE diff medium-I with or without test compound. Vortex plate at 500 rpm for 3 min. Leave the plate for 24 hours.

### Day 1. Change to Diff Media-II with drug

Wash plates three times with Advance RPMI quickly (∼10 min). Add DE diff medium-II with or without compound. Vortex plate at 500 rpm for 3 min. Leave the plate for 48 hours. Note that cells were treated with drugs for a total of 72 hours.

### Day 3. STOP AND FIX CELLS

1. Remove medium from 96 well plate. Wash well with DPBS GENTLY three times to remove debris or dead cells on top. Add freshly prepared 3%
   Formaldehyde in PBS which was pre-warmed at 37°C. Add this on cells for 15 min. Long fixation may cause high background or antigen alteration. Wash with PBS three times to remove PBS.
2. Permeabilize cells with PBS with 0.1% TritonX-100 for 15 min.
3. Apply 30ul ImageIT-FX solution per 96 well. Vortex at 700 rpm for 1 min. Incubate for 30 minutes at room temperature.
4. Wash with Blocking Buffer (PBS with 10% donkey serum, 0.3% Triton X-100, 1% BSA) once, then add Blocking Buffer, leave 20 min.
5. Add Sox17 antibody (1:10 dilution) in Blocking Buffer. Incubate for 3 hours at room temp.
6. Wash with PBS with 1% BSA four times.
7. Add PBS with 1% BSA with DAPI or Hoechst 33242 (5ug/ml final conc for both) for 5 min. If you use SlowFade with DAPI later, this procedure is not needed.
8. Add SlowFade with DAPI 30ul to each 96 well.

### Data Analysis

Measure the fluorescent intensity at 557 nm by Envision Plate reader.

### Protocol and Performance Criteria

The preceding example illustrates the invention as applied to the reference human embryonic stem cell H9 (Wisconsin Alumi Research Foundation, WARF). When applying the method to other pluripotent stem cell lines, one must determine the typical variables effect line-dependent differentiation efficiency such as initial cell seeding density, the concentrations of growth factors in the media and the duration of the differentiation. During this optimization process described above, we monitored the efficiency of mesendoderm formation as well as the drug effect mediated by thalidomide to identify the optimal conditions for the prediction of teratogenic risk.

## Claims

1. A method of assessing the teratogenic risk of a compound comprising:
(1) contacting the compound with pluripotent stem cells that are being differentiated into mesendoderm under conditions that reproducibly achieve 40-60% mesendoderm formation in 3 days of differentiation;
(2) measuring the protein expression of one or more mesendodermal markers at day 3 of differentiation, wherein the one or more mesendodermal markers are selected from the group consisting of Sox17, EOMES, and T-brachyury;
(3) determining the IC50 concentration of the compound which results in 50% inhibition of the protein expression of said one or more markers; and
(4) comparing the IC50 concentration determined in step (3) with the IC50 concentrations of:
(a) one or more compounds with known teratogenic risks and (b) one or more compounds with known non-teratogenic risks, to establish a teratogenic risk of the compound being assessed,
wherein the pluripotent stem cells are human pluripotent stem cells and wherein at least one of the mesendodermal markers is SOX17.

2. The method of any one of claim 1, wherein the pluripotent stem cells are induced pluripotent stem cells.

3. The method of any one of claims 1 or 2, wherein at least one of the mesendodermal markers is EOMES.

4. The method of any one of claims 1-3, wherein at least one of the mesendodermal markers is T-brachyury.

5. The method of claim 1, wherein the protein expression of SOX17 is the only mesendodermal marker measured.

6. The method of claim 1, wherein the protein expression of SOX17 and EOMES are the only mesendodermal markers measured.

7. The method of claim 1, wherein the protein expression of SOX17 and T-brachyury are the only mesendodermal markers measured.

8. The method of any one of claims 1-7, wherein the protein expression is measured by immuno-histochemistry.

9. The method of any one of claims 1-7, wherein the protein expression is measured by flow cytometry.

10. The method of any one of claims 1-7, wherein the protein expression is measured by fluorescence microscopy.

11. The method of any one of claims 1-10, wherein the one or more compounds with known teratogenic risks in step (4) are selected from the group consisting of: actinomycin D, tretinoin, cytarabine, nocodazole, rotenone, tretinoin, isotretinoin, bromodeoxyuridine, doxorubicin, dorsomorphin, thalidomide, 5-Fluorouracil, dasatinib, sorafenib, valproic acid, sunitinib, ziprasidone, mianserine, vandetanib, diethylstilbestrol, 6-amino-nicotinamide, ritanserin, and gefitinib.

12. The method of any one of claims 1-10, wherein the one or more compounds with known teratogenic risks in step (4) are selected from the group consisting of: thalidomide, mianserine, and ritanserin.

13. The method of any one of claims 1-12, wherein the one or more compounds with known non-teratogenic risks in step (4) are selected from the group consisting of: esomeprazole, folate, catechin, lisuride, niacin, aspirin, ibuprofen, acyclovir, ketanserine, streptomycin, methyldopa, saccharin, caffeine, penicillin, and tegaserod.

14. The method of any one of claims 1-12, wherein the one or more compounds with known non-teratogenic risks in step (4) are selected from the group consisting of: folate and methyldopa.

15. The method of any one of claims 1-10, wherein the one or more compounds with known teratogenic risks in step (4) are selected from the group consisting of: thalidomide, mianserine, and ritanserin; and the one or more compounds with known non-teratogenic risks are selected from the group consisting of folate and methyldopa.

## Patentansprüche

1. Verfahren zur Beurteilung des teratogenen Risikos einer Verbindung, umfassend:
(1) Inkontaktbringen der Verbindung mit pluripotenten Stammzellen, die sich unter Bedingungen, die innerhalb einer Differenzierung über 3 Tage reproduzierbar eine 40-60%ige Mesendodermbildung bewirken, zu Mesendoderm differenzieren;
(2) Messen der Proteinexpression von einem oder mehreren mesendodermalen Markern an Tag 3 der Differenzierung, wobei der eine oder die mehreren mesendodermalen Marker ausgewählt sind aus der Gruppe bestehend aus Soxl7, EOMES und T-Brachyurie;
(3) Bestimmen der IC50-Konzentration der Verbindung, die zu einer Hemmung der Proteinexpression des einen oder der mehreren Marker um 50 % führt; und
(4) Vergleichen der in Schritt (3) bestimmten IC50-Konzentration mit den IC50-Konzentrationen von:
(a) einer oder mehreren Verbindungen mit bekannten teratogenen Risiken und (b) einer oder mehreren Verbindungen mit bekannten nicht-teratogenen Risiken, um ein teratogenes Risiko der Verbindung, die beurteilt wird, festzustellen,
wobei die pluripotenten Stammzellen humane pluripotente Stammzellen sind und wobei mindestens einer der mesendodermalen Marker SOX17 ist.

2. Verfahren nach Anspruch 1, wobei die pluripotenten Stammzellen induzierte pluripotente Stammzellen sind.

3. Verfahren nach einem der Ansprüche 1 oder 2, wobei mindestens einer der mesendodermalen Marker EOMES ist.

4. Verfahren nach einem der Ansprüche 1-3, wobei mindestens einer der mesendodermalen Marker T-Brachyurie ist.

5. Verfahren nach Anspruch 1, wobei die Proteinexpression von SOX17 der einzige gemessene mesendodermale Marker ist.

6. Verfahren nach Anspruch 1, wobei die Proteinexpressionen von SOX17 und EOMES die einzigen gemessenen mesendodermalen Marker sind.

7. Verfahren nach Anspruch 1, wobei die Proteinexpressionen von SOX17 und T-Brachyurie die einzigen gemessenen mesendodermalen Marker sind.

8. Verfahren nach einem der Ansprüche 1-7, wobei die Proteinexpression durch Immunhistochemie gemessen wird.

9. Verfahren nach einem der Ansprüche 1-7, wobei die Proteinexpression durch Durchflusszytometrie gemessen wird.

10. Verfahren nach einem der Ansprüche 1-7, wobei die Proteinexpression durch Fluoreszenzmikroskopie gemessen wird.

11. Verfahren nach einem der Ansprüche 1-10, wobei die eine oder die mehreren Verbindungen mit bekannten teratogenen Risiken in Schritt (4) ausgewählt sind aus der Gruppe bestehend aus Actinomycin D, Tretinoin, Cytarabin, Nocodazol, Rotenon, Tretinoin, Isotretinoin, Bromdesoxyuridin, Doxorubicin, Dorsomorphin, Thalidomid, 5-Fluoruracil, Dasatinib, Sorafenib, Valproinsäure, Sunitinib, Ziprasidon, Mianserin, Vandetanib, Diethylstilbestrol, 6-Aminonicotinamid, Ritanserin und Gefitinib.

12. Verfahren nach einem der Ansprüche 1-10, wobei die eine oder die mehreren Verbindungen mit bekannten teratogenen Risiken in Schritt (4) ausgewählt sind aus der Gruppe bestehend aus Thalidomid, Mianserin und Ritanserin.

13. Verfahren nach einem der Ansprüche 1-12, wobei die eine oder die mehreren Verbindungen mit bekannten nicht-teratogenen Risiken in Schritt (4) ausgewählt sind aus der Gruppe bestehend aus Esomeprazol, Folat, Catechin, Lisurid, Niacin, Aspirin, Ibuprofen, Aciclovir, Ketanserin, Streptomycin, Methyldopa, Saccharin, Koffein, Penicillin und Tegaserod.

14. Verfahren nach einem der Ansprüche 1-12, wobei die eine oder die mehreren Verbindungen mit bekannten nicht-teratogenen Risiken in Schritt (4) ausgewählt sind aus der Gruppe bestehend aus Folat und Methyldopa.

15. Verfahren nach einem der Ansprüche 1-10, wobei die eine oder die mehreren Verbindungen mit bekannten teratogenen Risiken in Schritt (4) ausgewählt sind aus der Gruppe bestehend aus Thalidomid, Mianserin und Ritanserin; und die eine oder die mehreren Verbindungen mit bekannten nicht-teratogenen Risiken ausgewählt sind aus der Gruppe bestehend aus Folat und Methyldopa.

## Revendications

1. Procédé d'évaluation du risque tératogène d'un composé comprenant :
(1) la mise en contact du composé avec des cellules souches pluripotentes qui sont en cours de différenciation en mésendoderme dans des conditions qui conduisent de manière reproductible à la formation de 40 à 60% de mésendoderme en 3 jours de différenciation ;
(2) la mesure de l'expression protéique d'un ou de plusieurs marqueurs mésendodermiques au jour 3 de la différenciation, dans laquelle le ou les marqueurs mésendodermiques sont choisis dans le groupe constitué par le Sox17, l'EOMES et le T-brachyury ;
(3) la détermination de la concentration CI50 du composé qui entraîne une inhibition de 50 % de l'expression protéique dudit ou desdits marqueurs ; et
(4) la comparaison de la concentration CI50 déterminée à l'étape (3) avec les concentrations CI50 de :
(a) un ou plusieurs composés présentant des risques tératogènes connus et (b) un ou plusieurs composés présentant des risques non tératogènes connus, pour établir un risque tératogène du composé évalué,
dans lequel les cellules souches pluripotentes sont des cellules souches pluripotentes humaines et dans lequel au moins un des marqueurs mésendodermiques est le SOX17.

2. Procédé selon la revendication 1, dans lequel les cellules souches pluripotentes sont des cellules souches pluripotentes induites.

3. Procédé selon l'une quelconque des revendications 1 ou 2, dans lequel au moins un des marqueurs mésendodermiques est l'EOMES.

4. Procédé selon l'une quelconque des revendications 1 à 3, dans lequel au moins un des marqueurs mésendodermiques est le T-brachyury.

5. Procédé selon la revendication 1, dans lequel l'expression protéique de SOX17 est le seul marqueur mésendodermique mesuré.

6. Procédé selon la revendication 1, dans lequel les expressions protéiques de SOX17 et d'EOMES sont les seuls marqueurs mésendodermiques mesurés.

7. Procédé selon la revendication 1, dans lequel les expressions protéiques de SOX17 et de T-brachyury sont les seuls marqueurs mésendodermiques mesurés.

8. Procédé selon l'une quelconque des revendications 1 à 7, dans lequel l'expression protéique est mesurée par immunohistochimie.

9. Procédé selon l'une quelconque des revendications 1 à 7, dans lequel l'expression protéique est mesurée par cytométrie en flux.

10. Procédé selon l'une quelconque des revendications 1 à 7, dans lequel l'expression protéique est mesurée par microscopie de fluorescence.

11. Procédé selon l'une quelconque des revendications 1 à 10, dans lequel le ou les composés présentant des risques tératogènes connus à l'étape (4) sont choisis dans le groupe constitué par : l'actinomycine D, la trétinoïne, la cytarabine, le nocodazole, la roténone, la trétinoïne, l'isotrétinoïne, la bromodésoxyuridine, la doxorubicine, la dorsomorphine, le thalidomide, le 5-fluorouracile, le dasatinib, le sorafénib, l'acide valproïque, le sunitinib, la ziprasidone, la miansérine, le vandétanib, le diéthylstilbestrol, le 6-amino-nicotinamide, la ritansérine et le géfitinib.

12. Procédé selon l'une quelconque des revendications 1 à 10, dans lequel le ou les composés présentant des risques tératogènes connus à l'étape (4) sont choisis dans le groupe constitué par : le thalidomide, la miansérine et la ritansérine.

13. Procédé selon l'une quelconque des revendications 1 à 12, dans lequel le ou les composés présentant des risques non tératogènes connus à l'étape (4) sont choisis dans le groupe constitué par : l'ésoméprazole, le folate, la catéchine, le lisuride, la niacine, l'aspirine, l'ibuprofène, l'aciclovir, la kétansérine, la streptomycine, la méthyldopa, la saccharine, la caféine, la pénicilline et le tégasérod.

14. Procédé selon l'une quelconque des revendications 1 à 12, dans lequel le ou les composés présentant des risques non tératogènes connus à l'étape (4) sont choisis dans le groupe constitué par : le folate et la méthyldopa.

15. Procédé selon l'une quelconque des revendications 1 à 10, dans lequel le ou les composés présentant des risques tératogènes connus à l'étape (4) sont choisis dans le groupe constitué par : le thalidomide, la miansérine et la ritansérine ; et le ou les composés présentant des risques non tératogènes connus sont choisis dans le groupe constitué par le folate et la méthyldopa.


### SEQUENCE LISTING

<110> F. Hoffmann- La Roche AG
<120> METHOD OF DETERMINING TERATOGENIC RISK
<130> 30747 WO
<150> US 61/607622
   <151> 2012-03-07
<150> US 61/668489
   <151> 2012-07-06
<160> 4
<170> PatentIn version 3.5
<210> 1
   <211> 414
   <212> PRT
   <213> Homo sapiens
<400> 1
<210> 2
   <211> 686
   <212> PRT
   <213> Homo sapiens
<400> 2
   Met Gln Leu Gly Glu Gln Leu Leu Val Ser Ser Val Asn Leu Pro Gly
   1               5                   10                  15
   Ala His Phe Tyr Pro Leu Glu Ser Ala Arg Gly Gly Ser Gly Gly Ser
               20                  25                  30
   Ala Gly His Leu Pro Ser Ala Ala Pro Ser Pro Gln Lys Leu Asp Leu
           35                  40                  45
   Asp Lys Ala Ser Lys Lys Phe Ser Gly Ser Leu Ser Cys Glu Ala Val
       50                  55                  60
   Ser Gly Glu Pro Ala Ala Ala Ser Ala Gly Ala Pro Ala Ala Met Leu
   65                  70                  75                  80
   Ser Asp Thr Asp Ala Gly Asp Ala Phe Ala Ser Ala Ala Ala Val Ala
                   85                  90                  95
   Lys Pro Gly Pro Pro Asp Gly Arg Lys Gly Ser Pro Cys Gly Glu Glu
               100                 105                 110
   Glu Leu Pro Ser Ala Ala Ala Ala Ala Ala Ala Ala Ala Ala Ala Ala
           115                 120                 125
   Ala Ala Thr Ala Arg Tyr Ser Met Asp Ser Leu Ser Ser Glu Arg Tyr
       130                 135                 140
   Tyr Leu Gln Ser Pro Gly Pro Gln Gly Ser Glu Leu Ala Ala Pro Cys
   145                 150                 155                 160
   Ser Leu Phe Pro Tyr Gln Ala Ala Ala Gly Ala Pro His Gly Pro Val
                   165                 170                 175
   Tyr Pro Ala Pro Asn Gly Ala Arg Tyr Pro Tyr Gly Ser Met Leu Pro
               180                 185                 190
   Pro Gly Gly Phe Pro Ala Ala Val Cys Pro Pro Gly Arg Ala Gln Phe
           195                 200                 205
   Gly Pro Gly Ala Gly Ala Gly Ser Gly Ala Gly Gly Ser Ser Gly Gly
           210                 215                 220
   Gly Gly Gly Pro Gly Thr Tyr Gln Tyr Ser Gln Gly Ala Pro Leu Tyr
   225                 230                 235                 240
   Gly Pro Tyr Pro Gly Ala Ala Ala Ala Gly Ser Cys Gly Gly Leu Gly
                   245                 250                 255
   Gly Leu Gly Val Pro Gly Ser Gly Phe Arg Ala His Val Tyr Leu Cys
               260                 265                 270
   Asn Arg Pro Leu Trp Leu Lys Phe His Arg His Gln Thr Glu Met Ile
           275                 280                 285
   Ile Thr Lys Gln Gly Arg Arg Met Phe Pro Phe Leu Ser Phe Asn Ile
       290                 295                 300
   Asn Gly Leu Asn Pro Thr Ala His Tyr Asn Val Phe Val Glu Val Val
   305                 310                 315                 320
   Leu Ala Asp Pro Asn His Trp Arg Phe Gln Gly Gly Lys Trp Val Thr
                   325                 330                 335
   Cys Gly Lys Ala Asp Asn Asn Met Gln Gly Asn Lys Met Tyr Val His
               340                 345                 350
   Pro Glu Ser Pro Asn Thr Gly Ser His Trp Met Arg Gln Glu Ile Ser
           355                 360                 365
   Phe Gly Lys Leu Lys Leu Thr Asn Asn Lys Gly Ala Asn Asn Asn Asn
       370                 375                 380
   Thr Gln Met Ile Val Leu Gln Ser Leu His Lys Tyr Gln Pro Arg Leu
   385                 390                 395                 400
   His Ile Val Glu Val Thr Glu Asp Gly Val Glu Asp Leu Asn Glu Pro
                   405                 410                 415
   Ser Lys Thr Gln Thr Phe Thr Phe Ser Glu Thr Gln Phe Ile Ala Val
               420                 425                 430
   Thr Ala Tyr Gln Asn Thr Asp Ile Thr Gln Leu Lys Ile Asp His Asn
           435                 440                 445
   Pro Phe Ala Lys Gly Phe Arg Asp Asn Tyr Asp Ser Ser His Gln Ile
       450                 455                 460
   Val Pro Gly Gly Arg Tyr Gly Val Gln Ser Phe Phe Pro Glu Pro Phe
   465                 470                 475                 480
   Val Asn Thr Leu Pro Gln Ala Arg Tyr Tyr Asn Gly Glu Arg Thr Val
                   485                 490                 495
   Pro Gln Thr Asn Gly Leu Leu Ser Pro Gln Gln Ser Glu Glu Val Ala
               500                 505                 510
   Asn Pro Pro Gln Arg Trp Leu Val Thr Pro Val Gln Gln Pro Gly Thr
           515                 520                 525
   Asn Lys Leu Asp Ile Ser Ser Tyr Glu Ser Glu Tyr Thr Ser Ser Thr
       530                 535                 540
   Leu Leu Pro Tyr Gly Ile Lys Ser Leu Pro Leu Gln Thr Ser His Ala
   545                 550                 555                 560
   Leu Gly Tyr Tyr Pro Asp Pro Thr Phe Pro Ala Met Ala Gly Trp Gly
                   565                 570                 575
   Gly Arg Gly Ser Tyr Gln Arg Lys Met Ala Ala Gly Leu Pro Trp Thr
               580                 585                 590
   Ser Arg Thr Ser Pro Thr Val Phe Ser Glu Asp Gln Leu Ser Lys Glu
           595                 600                 605
   Lys Val Lys Glu Glu Ile Gly Ser Ser Trp Ile Glu Thr Pro Pro Ser
       610                 615                 620
   Ile Lys Ser Leu Asp Ser Asn Asp Ser Gly Val Tyr Thr Ser Ala Cys
   625                 630                 635                 640
   Lys Arg Arg Arg Leu Ser Pro Ser Asn Ser Ser Asn Glu Asn Ser Pro
                   645                 650                 655
   Ser Ile Lys Cys Glu Asp Ile Asn Ala Glu Glu Tyr Ser Lys Asp Thr
               660                 665                 670
   Ser Lys Gly Met Gly Gly Tyr Tyr Ala Phe Tyr Thr Thr Pro
           675                 680                 685
<210> 3
   <211> 435
   <212> PRT
   <213> Homo sapiens
<400> 3
   Met Ser Ser Pro Gly Thr Glu Ser Ala Gly Lys Ser Leu Gln Tyr Arg
   1               5                   10                  15
   Val Asp His Leu Leu Ser Ala Val Glu Asn Glu Leu Gln Ala Gly Ser
               20                  25                  30
   Glu Lys Gly Asp Pro Thr Glu Arg Glu Leu Arg Val Gly Leu Glu Glu
           35                  40                  45
   Ser Glu Leu Trp Leu Arg Phe Lys Glu Leu Thr Asn Glu Met Ile Val
       50                  55                  60
   Thr Lys Asn Gly Arg Arg Met Phe Pro Val Leu Lys Val Asn Val Ser
   65                  70                  75                  80
   Gly Leu Asp Pro Asn Ala Met Tyr Ser Phe Leu Leu Asp Phe Val Ala
                   85                  90                  95
   Ala Asp Asn His Arg Trp Lys Tyr Val Asn Gly Glu Trp Val Pro Gly
               100                 105                 110
   Gly Lys Pro Glu Pro Gln Ala Pro Ser Cys Val Tyr Ile His Pro Asp
           115                 120                 125
   Ser Pro Asn Phe Gly Ala His Trp Met Lys Ala Pro Val Ser Phe Ser
       130                 135                 140
   Lys Val Lys Leu Thr Asn Lys Leu Asn Gly Gly Gly Gln Ile Met Leu
   145                 150                 155                 160
   Asn Ser Leu His Lys Tyr Glu Pro Arg Ile His Ile Val Arg Val Gly
                   165                 170                 175
   Gly Pro Gln Arg Met Ile Thr Ser His Cys Phe Pro Glu Thr Gln Phe
               180                 185                 190
   Ile Ala Val Thr Ala Tyr Gln Asn Glu Glu Ile Thr Ala Leu Lys Ile
           195                 200                 205
   Lys Tyr Asn Pro Phe Ala Lys Ala Phe Leu Asp Ala Lys Glu Arg Ser
       210                 215                 220
   Asp His Lys Glu Met Met Glu Glu Pro Gly Asp Ser Gln Gln Pro Gly
   225                 230                 235                 240
   Tyr Ser Gln Trp Gly Trp Leu Leu Pro Gly Thr Ser Thr Leu Cys Pro
                   245                 250                 255
   Pro Ala Asn Pro His Pro Gln Phe Gly Gly Ala Leu Ser Leu Pro Ser
               260                 265                 270
   Thr His Ser Cys Asp Arg Tyr Pro Thr Leu Arg Ser His Arg Ser Ser
           275                 280                 285
   Pro Tyr Pro Ser Pro Tyr Ala His Arg Asn Asn Ser Pro Thr Tyr Ser
       290                 295                 300
   Asp Asn Ser Pro Ala Cys Leu Ser Met Leu Gln Ser His Asp Asn Trp
   305                 310                 315                 320
   Ser Ser Leu Gly Met Pro Ala His Pro Ser Met Leu Pro Val Ser His
                   325                 330                 335
   Asn Ala Ser Pro Pro Thr Ser Ser Ser Gln Tyr Pro Ser Leu Trp Ser
               340                 345                 350
   Val Ser Asn Gly Ala Val Thr Pro Gly Ser Gln Ala Ala Ala Val Ser
           355                 360                 365
   Asn Gly Leu Gly Ala Gln Phe Phe Arg Gly Ser Pro Ala His Tyr Thr
       370                 375                 380
   Pro Leu Thr His Pro Val Ser Ala Pro Ser Ser Ser Gly Ser Pro Leu
   385                 390                 395                 400
   Tyr Glu Gly Ala Ala Ala Ala Thr Asp Ile Val Asp Ser Gln Tyr Asp
                   405                 410                 415
   Ala Ala Ala Gln Gly Arg Leu Ile Ala Ser Trp Thr Pro Val Ser Pro
               420                 425                 430
   Pro Ser Met
           435
<210> 4
   <211> 360
   <212> PRT
   <213> Homo sapiens
<400> 4
   Met Ala Gly His Leu Ala Ser Asp Phe Ala Phe Ser Pro Pro Pro Gly
   1               5                   10                  15
   Gly Gly Gly Asp Gly Pro Gly Gly Pro Glu Pro Gly Trp Val Asp Pro
               20                  25                  30
   Arg Thr Trp Leu Ser Phe Gln Gly Pro Pro Gly Gly Pro Gly Ile Gly
           35                  40                  45
   Pro Gly Val Gly Pro Gly Ser Glu Val Trp Gly Ile Pro Pro Cys Pro
       50                  55                  60
   Pro Pro Tyr Glu Phe Cys Gly Gly Met Ala Tyr Cys Gly Pro Gln Val
   65                  70                  75                  80
   Gly Val Gly Leu Val Pro Gln Gly Gly Leu Glu Thr Ser Gln Pro Glu
                   85                  90                  95
   Gly Glu Ala Gly Val Gly Val Glu Ser Asn Ser Asp Gly Ala Ser Pro
               100                 105                 110
   Glu Pro Cys Thr Val Thr Pro Gly Ala Val Lys Leu Glu Lys Glu Lys
           115                 120                 125
   Leu Glu Gln Asn Pro Glu Glu Ser Gln Asp Ile Lys Ala Leu Gln Lys
       130                 135                 140
   Glu Leu Glu Gln Phe Ala Lys Leu Leu Lys Gln Lys Arg Ile Thr Leu
   145                 150                 155                 160
   Gly Tyr Thr Gln Ala Asp Val Gly Leu Thr Leu Gly Val Leu Phe Gly
                   165                 170                 175
   Lys Val Phe Ser Gln Thr Thr Ile Cys Arg Phe Glu Ala Leu Gln Leu
               180                 185                 190
   Ser Phe Lys Asn Met Cys Lys Leu Arg Pro Leu Leu Gln Lys Trp Val
           195                 200                 205
   Glu Glu Ala Asp Asn Asn Glu Asn Leu Gln Glu Ile Cys Lys Ala Glu
       210                 215                 220
   Thr Leu Val Gln Ala Arg Lys Arg Lys Arg Thr Ser Ile Glu Asn Arg
   225                 230                 235                 240
   Val Arg Gly Asn Leu Glu Asn Leu Phe Leu Gln Cys Pro Lys Pro Thr
                   245                 250                 255
   Leu Gln Gln Ile Ser His Ile Ala Gln Gln Leu Gly Leu Glu Lys Asp
               260                 265                 270
   Val Val Arg Val Trp Phe Cys Asn Arg Arg Gln Lys Gly Lys Arg Ser
           275                 280                 285
   Ser Ser Asp Tyr Ala Gln Arg Glu Asp Phe Glu Ala Ala Gly Ser Pro
       290                 295                 300
   Phe Ser Gly Gly Pro Val Ser Phe Pro Leu Ala Pro Gly Pro His Phe
   305                 310                 315                 320
   Gly Thr Pro Gly Tyr Gly Ser Pro His Phe Thr Ala Leu Tyr Ser Ser
                   325                 330                 335
   Val Pro Phe Pro Glu Gly Glu Ala Phe Pro Pro Val Ser Val Thr Thr
               340                 345                 350
   Leu Gly Ser Pro Met His Ser Asn
           355                 360